# FASCICULE DE BREVET EUROPEEN

(11) **EP 1 007 004 B1**
(45) Date de publication et mention de la délivrance du brevet: **12.09.2001**
(21) Numéro de dépôt: 98940323.3
(22) Date de dépôt: 23.07.1998
(51) Int. Cl.: A61K 9/16, A61K 9/52

(54) **PROCEDE POUR LA PREPARATION D'UN EXTRAIT DE PRINCIPES ACTIFS SOUS FORME DE MICROGRANULES A BASE DE FIBRES ALIMENTAIRES**
VERFAHREN ZUR HERSTELLUNG EINES WIRKSTOFFEXTRAKTS IN FORM VON MIKROGRANULATEN AUF BASIS VON NAHRUNGSFASERN
METHOD FOR PREPARING AN EXTRACT OF ACTIVE PRINCIPLES IN THE FORM OF MICROGRANULES BASED ON DIETARY FIBRES

(30) Priorité: 31.07.1997 FR 9710041
(43) Date de publication de la demande: 14.06.2000
(73) Titulaire: Vegetalys Corp., Tortola (VG)
(72) Inventeur: CINGOTTI, Dominique, 69100 Villeurbanne (FR)
(74) Mandataire: Vuillermoz, Bruno
(86) Numéro de dépôt international: FR9801634
(87) Numéro de publication internationale: WO9906028

(56) Documents cités:
- EP-A- 0 498 463
- EP-A- 0 524 484
- EP-A- 0 686 399
- GB-A- 2 041 220

## Description

L'invention concerne un nouveau procédé de préparation de microgranulés entièrement solubles, préparés à partir d'extraits ou de solutions de principes actifs; ces microgranulés sont destinés à être utilisés tels que pour la reconstitution de solutions ou à être incorporés dans différentes formes galéniques.

L'administration et l'utilisation d'extraits de principes actifs, surtout d'origine naturelle, sous forme d'Huiles Essentielles, de solutions d'extraction par solvants posent des problèmes à différents niveaux lors de l'utilisation :
- mise en solution incomplète
- difficulté de dosage précis
- masquage de goût
- présentation galénique inadaptée

L'obtention sous forme sèche de ces différents extraits de principes actifs, fait appel pour la plupart, à des procédés utilisant un apport calorifique important; nebulisation-atomisation, évaporation sous vide ou non, reprise de broyage ou de micronisation, ces différentes opérations pouvant apporter des altérations substantielles. Les formes sèches ainsi obtenues de ces différents extraits sont soumises à des altérations de principes actifs, liées à une forte hygroscopicité du fait des augmentations de surface spécifique induites par les traitements physiques cités.

On a par exemple proposé dans le document EP-A-0 524 484 une poudre pour préparation de boissons instantanées, obtenue par dissolution aqueuse d'extraits de thé et d'un support d'inuline, le mélange étant ensuite séché, puis atomisé.

EP-A-0 686 395 décrit un procédé pour la préparation de grains d'amidon, comportant une importante surface poreuse, qui permettent d'absorber des solutions aqueuses, des huiles ou des composants en milieu solvant.

Même si on obtient une composition de faibles calories de part la dégradation du support en unités fructoses, la préparation de celle-ci nécessite un volume important d'inuline pour une quantité donnée d'extrait, contribuant à augmenter le coût de la formulation. En outre, l'énergie thermique dégagée lors de la phase d'atomisation de la poudre une fois que le principe actif est fixé sur l'inuline conduit à dégrader thermiquement ledit principe actif, ce qui exclut la mise en oeuvre de principes actifs thermosensibles. Par ailleurs, le procédé mis en oeuvre exclut l'incorporation de toute substance insoluble dans l'eau, notamment les substances volatiles, en particulier les arômes et les huiles essentielles, qui seront évaporées partiellement ou totalement lors du processus d'atomisation.

Le problème que se propose de résoudre l'invention est donc de développer un procédé de fabrication d'un extrait de principe actif d'un coût réduit en cherchant à optimiser les proportions de chacun des constituants.

Un autre objectif de l'invention est d'assurer une stabilité dans le temps de la préparation fabriquée en mettant en oeuvre un procédé sans dégradation thermique permettant d'incorporer des principes actifs ou des substances d'extraction thermosensibles.

L'invention a également pour but de proposer un procédé de fabrication d'un extrait de principe actif dans lequel on ne soit pas limité par le choix du solvant, permettant ainsi de mettre en oeuvre tout type de principe actif indépendamment de ses caractéristiques physico-chimiques.

Pour résoudre l'ensemble de ces problèmes, l'invention propose un procédé pour la préparation d'un extrait d'au moins un principe actif sous la forme de granulés secs entièrement solubles , qui consiste :
- tout d'abord, à préparer un extrait de principe actif dans un milieu solvant ;
- puis, à déposer cet extrait sur les parois internes et externes d'un support microporeux non soluble dans le milieu solvant, se présentant sous forme de microgranules constitués d'une fibre alimentaire soluble, à base d'un polymère choisi dans le groupe comprenant l'inuline et les oligofructoses ou fructo-oligosaccharides, seuls ou en mélange, lesdits microgranules ayant une surface spécifique d'au moins 0,50 m²/g ;
- et à sécher le support granulé imprégné obtenu ;
- puis, à recouvrir les parois imprégnées du support microporeux par un polymère filmogène.

En d'autres termes, l'invention consiste :
- dans un premier temps, à préparer des extraits d'origine végétale, animale ou autre, ou des solutions de principes actifs ;
- dans un second temps, à imprégner les microgranules dont la surface spécifique d'au moins 0,50 m²/g assure une imprégnation optimum, ces microgranules étant insolubles dans les extraits ou solutions de principes actifs ;
- dans un troisième temps, à évaporer par séchage le solvant, les substances de l'extrait ou de la solution de principes actifs restant absorbées dans la structure microscopique du support microgranulé ;
- et enfin, à enrober le microgranulé obtenu, par un pelliculage de polymères lui permettant d'assurer une stabilité dans le temps au sein de la forme galénique qui le véhiculera lors de son administration, et aussi de pouvoir assurer une libération progressive des principes actifs retenus, à travers le film de polymères.

Dans la description et dans les revendications, par "extraits ou solutions de principes actifs", il faut entendre toute forme de solution contenant des principes actifs d'origine végétale animale ou autre, telles que solutions de principes actifs de synthèse et d'origine minérale (oligoéléments ...), macérations, huiles essentielles, gommes, résines. On prépare ces extraits de manière connue, notamment dans un milieu solvant approprié aux principes actifs recherchés, ou par un procédé physique spécifique soit respectivement l'éthanol pour l'extrait, ou la distillation par entraînement à la vapeur d'eau ( huiles essentielles, substances volatiles). En fonction des principes actifs, on utilise comme solvant, un solvant qui solubilise les dits principes actifs et qui respecte la structure du support microgranulé, et qui ne présente pas de toxicité. En pratique, on fait appel à l'éthanol. On peut rajouter à l'extrait ou à la solution de principes actifs des adjuvants dans le but de faciliter la dissolution, la dispersion des principes actifs lors de la fabrication, ou d'assurer une protection mécanique du produit final.

Dans la description et dans les revendications , par "Fibres alimentaires", on désigne tout composé de cellules végétales, résistant aux différentes hydrolyses enzymatiques, mises en oeuvre lors du processus physiologique de la digestion. Ces fibres se composent de cellulose, d'hémicellulose, d'oligosaccharides, pectine, gommes, cires, et de lignine, ces différents composés sont ceux pris en compte dans les analyses de fibres alimentaires.

En pratique :
- le principe actif est un extrait de plantes ou de partie de plantes ;
- le milieu solvant est constitué par un solvant du groupe comprenant l'eau, l'éthanol, l'acétone, le dichlorométhane ;
- le support microporeux est un microgranulé obtenu par atomisation ou par granulation par voie humide ;
- le microgranulé atomisé est à base de fibres solubles pouvant être associé à d'autres substances comme des polyalcools (sorbitol, xylitol, mannitol), des polyols ( maltodextrines ...), ou des corps gras ;
- le dépôt de l'extrait sur les parois est effectué par granulation humide et le séchage est effectué à l'air chaud ;
- le polymère filmogène est un composé soluble dans le système digestif gastrosoluble (hydroxypropylméthylcellulose) ou gastrorésistant (phtalate d'hydroxypropylméthylcellulose);
- le polymère filmogène est pulvérisé en turbine ou en lit d'air fluidisé.

Une fois cet extrait ou solution de principes actifs préparé de manière connue, on prépare le microgranulé par la technique d'atomisation ou granulation par voie humide, qui permet d'obtenir un support microgranulé de structure poreuse quantifiée par sa surface spécifique : unité de surface /unité de masse ; généralement on utilise comme fibres alimentaires des fibres solubles, tel que l'inuline et les oligofructoses, qui sont des substances végétales naturelles, permettant le stockage des hydrates de carbones dans différentes plantes, entrant dans l'alimentation humaine, telles que les poireaux, l'oignon, le froment, l'ail, la chicorée, l'artichaut etc ...

Selon une première forme de réalisation de l'invention, le support microgranulé est une poudre à base d'inuline obtenue par extraction à partir de racines de chicorée ou de tout autre végétal riche en inuline .

L'inuline est un melange d'oligomères et de polymères de fructose, dont la structure est représentée par la formule GFn, dans laquelle :
G : unité glucosyl
F : unité fructosyl
n : nombre d'unités fructosyl liées (n supérieur à 2)
DP : degré de polymérisation variant de 2 à 60
Liaison du type β (1-2)

Selon une seconde forme de réalisation de l'invention, le support microgranulé est une poudre à base d 'oligofructoses obtenus par hydrolyse de l'inuline. De même, les oligofructoses ou fructo-oligosaccharides peuvent être obtenus par synthèse.

Les oligofructoses ou fructo-oligosaccharides sont un mélange d'oligomères et de polymères de fructose, dont la structure est représentée par la formule GFn ou Fm , dans laquelle :
G : unité glucosyl
F : unité fructosyl
n et m : nombre d'unités fructosyl liées (n supérieur à 2)
DP : degré de polymérisation variant de 2 à 20
Les oligofructoses contiennent des molécules du type GFn et du type Fm
Liaison du type β (1-2)

La surface spécifique du microgranulé devra être au moins supérieure ou égale à 0,50 m² /Gramme de support.

Ce support poreux microgranulé est bien connu et largement utilisé, ce qui fait qu'on peut le trouver disponible prêt à l'emploi.

On peut additionner d'autres substances préparées sous forme microgranulés par la même technique d'atomisation que les fibres ci-dessus :
Sucres
dérivés de sucres : hydrolysats esters, alcools, autres
dérivés lipophiles, cires, polymères, autres
Polysaccharides et Hydrolysats
Protéïnes

La surface spécifique du microgranulé devra être au moins supérieure ou égale à 0,50 m²/g de support.

Ce support poreux microgranulé est bien connu et largement utilisé, ce qui fait qu'on peut le trouver disponible prêt à l'emploi.

Le dépôt s'effectue aux conditions normales de température et de pression , au sein d'un mélangeur granulateur conventionnel (Planétaire, à Rubans, Socs de charrue) ou d'un granulateur à lit d'air fluidisé. On place tout d'abord le support microgranulé au sein du mélangeur que l'on mouille par l'extrait, à raison de 1% à 60% de la masse. En effet, si le volume de l'extrait excède cette limite de 60%, on obtient une suspension de microgranules, la strucure du microgranule ne pouvant plus absorber l'extrait.

Une fois le support imprégné jusqu'à 60 % maximum, on sèche le support imprégné sous atmosphère ventilée, à température moyenne, par exemple 50°C, avec recyclage et récupération éventuelle de solvant. Il est à noter que si l'on utilise un granulateur à lit d'air fluidisé, on effectue de façon simultanée l'imprégnation et le séchage du support. Une fois le séchage terminé, on calibre le microgranulé de façon à éliminer les particules inférieures à la taille moyenne.

Une fois les microgranulés obtenus et pour des raisons de conservation, d'administration et libération des principes actifs dans l'organisme, ces microgranulés peuvent subir une opération complémentaire d'enrobage par film de polymères, utilisant les moyens conventionnels (turbine, lit d'air fluidisé ...).

Les principaux polymères filmogènes utilisés en enrobage peuvent être classés en fonction de leur solubilité et de la biodisponibilité qu'ils procurent aux microgranulés enrobés. En polymères gastrosolubles (hydroxypropylcellullose...) en polymères gastrorésistants (acétylphtalate de Cellulose, phtalate d'hydroxypropyl de cellulose...), et en polymères insolubles (éthyl cellulose ...) permettant la réalisation d'enrobages à libération prolongée. Ces polymères filmogènes sont généralement employés en milieu organique, bien que dans chaque catégorie de polymères certains d'entre eux soient également utilisés en milieu aqueux quand le support microgranulé le permet. L'enrobage se fait à partir de la solution choisie de polymères à laquelle on incorpore ci besoin des additifs tels que plastifiants et de charge couramment employés :
lubrifiants, opacifiants
pigments colorants

Avantageusement, la quantité de support microgranulé peut absorber 5 à 60% de volume de solution d'enrobage par rapport à 100% du poids du support, sachant que la solution d'enrobage composée d'extraits ou de solution de principes actifs peut contenir des p.p.m. à 20% de résidu sec. En revanche, quand le rapport volume/poids excède 60%, on note une destruction du support microgranulé et une perte de résidu sec. En effet, la solution ne pouvant être absorbée par le support microgranulé, la solution et à fortiori son résidu sec restent sur les parois de l'appareillage avec une perte d'actifs sur le produit final, et avec des difficultés de sèchage dans le procédé. Comme déjà dit, on obtient d'excellents résultats avec les proportions de 5 à 60 % de volume de solution d'enrobage par rapport à 100 % du poids d'excipient soit 5 ml à 60 ml de solution d'enrobage pour 100 g d'excipient microgranulé.

Comme support microgranulé, on fait appel de préférence à des fibres alimentaires solubles, pouvant être associées à des substances du type ; Polyalcools, Gommes, Mucilages, Résines, Protéïnes, etc..obtenus par nébulisation donnant une surface spécifique à l'unité de poids nécessaire. Ce type d'opération donne au support une capacité d'absorption vis à vis de la solution d'extraits ou de principes actifs, et assure, après élimination du solvant de la solution, une rétention de son résidu sec (contenant les actifs ) dans la structure du support microgranulé. Ces microgranulés absorbés peuvent être mis en solution tels que, ou être intégrés dans différentes formes galéniques, gélules, comprimés, formes pâteuses continues (hydrophiles-lipophiles ) ou émulsions ...

L'utilisation de fibres alimentaires solubles permet de mettre en valeur leurs caractéristiques intrinsèques, qui sont :
- L'inuline et les oligofructoses qui sont des fibres alimentaires, ne sont pas hydrolysées, de manière significative lors du passage en milieu acide dans l'estomac. Les enzymes de l'organisme présentes dans l'intestin grêle, du type sucrase-maltase , abondamment présentes dans l'intestin, n'ont aucune action d'hydrolyse sur les liaisons des unités formant ces fibres, ne provoquant aucune augmentation de la glycémie, et du taux d'insuline dans le sang. De la sorte, ces fibres peuvent être utilisées, dans le régime destiné aux diabétiques.
- L'inuline et les oligofructoses, au niveau du côlon, sont rapidement fermentées par la flore intestinale Les hydrates de carbone sont métabolisés en acides gras avec un faible nombre de carbone (acide propionique, acide lactique, acide butyrique, etc ...), et en gaz.

L'utilisation de fibres alimentaires solubles comme support, dont la dégradation intervient en majorité dans la phase finale de la digestion, au niveau du gros intestin et du côlon, du fait de la résistance à l'hydrolyse enzymatique des fibres, présente les avantages suivants :
- Le processus de désagrégation contrôlée, fibres metabolisées "in fine", permet d'obtenir une liaison principes actifs-fibres présente tout au long du cursus d'assimilation digestive, consolidée par l'enrobage de films de polymères, apportant ainsi une biodisponibilité accrue, et spécifique.
- Faible pouvoir calorifique :
   inuline 1,0 kcal /g contre 4,0 kcal/g pour les glucides
   inuline 1,0 kcal /g contre 2,40kcal/g pour les polyols
   oligofructose 1,5 kcal /g contre 4,0 kcal/g pour les glucides
   oligofructose 1,5 kcal /g contre 2,40kcal/g pour les polyols
- La faible dégradation de ces fibres, n'apportant aucune réponse de la glycémie et de l'insuline dans le sang, fait de ces fibres un support spécifique dans la diétique destinée aux pathologies diabétiques.
- Stimulation du bifidus : la dégradation dans le côlon des fibres stimule le métabolisme des bifidobactéries

On obtient donc des microgranules utilisables dans diverses formes galéniques, qui présentent une structure réticulaire corrélée par leur surface spécifique/unité de poids, ce réseau réticulaire étant tapissé par le résidu sec ou les principes actifs qui étaient présents dans la solution absorbée. Lesdits microgranulés absorbés peuvent être enrobés par un film de polymères, assurant des propriétés de stabilité, de solubilité, de biodisponibilité spécifiques.

La manière dont l'invention peut être réalisée et les avantages qui en découlent ressortiront mieux dans les exemples de réalisation qui suivent.

### EXEMPLE N°1 : Préparation d'une boisson phytothérapique à visée amaigrissante entièrement soluble.

De manière connue, on prépare un extrait alcoolique de fucus-frêne-romarin, par macération dans de l'éthanol à 80° .

Après le temps de macération (par exemple 3 semaines), on soutire, on essore, on filtre. Cet extrait hydroalcoolique a une composition approximative pour 100 ml :

| | |
|---|---|
| polyphénols totaux : | 0,80 g |
| iode | 3,00 mg |
| mucilages | 0,10 g |

On prépare 2 supports microgranulés de façon à obtenir une surface spécifique supérieure à 0,5 m² /gramme par nébulisation (lors de la nébulisation, il peut être nécessaire d'effectuer une microgranulation)
- 1 er support obtenu par atomisation d'une solution d'inuline à 95 % (on peut utiliser un support inuline prêt à l'emploi obtenu de la même façon : RAFTILINE GR (ORAFTI) avec une granulométrie moyenne de 0,30 mm)
- 2 ème support obtenu par atomisation d'une solution de sorbitol (on peut utiliser un support sorbitol prêt à l'emploi obtenu de la même façon : SORBITOL INSTANT (MERCK) avec une granulométrie moyenne de 0,50 mm).

Ces microgranulés sont placés à raison de 75 % inuline et 25 % sorbitol dans un mélangeur à température ambiante, et l'on effectue la granulation des microgranulés avec la solution d'extrait, à raison de 50 % de la masse (50ml/100g) et une incorporation de gomme de guar à 2,50 %.

Le microgranulé ainsi imprégné est mis à séché en étuve à 40 °C ; on obtient après séchage, des microgranulés porteurs de l'extrait sec de couleur verte. Puis on calibre de façon à rétablir la granulométrie de départ.

On obtient donc des microgranulés d'inuline et de sorbitol, dont les cavités internes et la surface externe sont recouvertes de l'extrait sec de la solution alcoolique obtenue par macération, et de gomme de guar. Ladite gomme de guar, incorporée lors de la granulation, se colle à l'intérieur des cavités internes et à la surface du microgranulé .

On recouvre ensuite les microgranulés obtenus, par pulvérisation d'une solution (à 10 %) d'acétyl phtalate de cellulose dans le dichlorométhane ; la pulvérisation se fait en turbine avec pulvérisation d'air chaud, assurant la formation du film. Le dit film est soluble à un pH > 5 correspondant aux conditions de mise en solution lors de l'utilisation.

Les microgranulés ainsi obtenus, permettent de reconstituer une forme à dissolution instantanée, pour préparation de boisson à visée amaigrissante . On obtient donc une forme galénique, équivalente à une tisane effectuée avec les mêmes espèces végétales, et qui comporte les avantages suivants :
Microgranulés enrobés par film, assurant la protection et la stabilité des principes actifs, lors du stockage
Dissolution instantanée
Préparation de la boisson : Eau froide ou Eau chaude, conservant les mêmes principes actifs en quantité et en qualité
Présentation commerciale attractive

On répète cet exemple , en modifiant le 2ème support cité ci dessus :
- 2 ème support obtenu par atomisation d'une solution de maltodextrine (on peut utiliser un support maltodextrine prêt à l'emploi obtenu de la même façon : GLUCIDEX IT 19 (ROQUETTE) avec une granulométrie moyenne de 0,30 mm) ;
- dans les mêmes conditions que précédemment, on obtient un microgranulé. présentant les mêmes caractéristiques.

### EXEMPLE N°2: Préparation d'une boisson phytothérapique à visée laxative entièrement soluble.

De manière connue, on prépare un extrait alcoolique (A) d'un mélange de plantes : réglisse-anis-romarin par macération dans de l'éthanol à 96°.

Après le même temps de macération comme à l'exemple 1, on soutire , on essore, on filtre cet extrait alcoolique qui assurera l'aromatisation .

De manière connue, on prépare un extrait aqueux de feuilles de senné ( B ), de façon à extraire les principes actifs (sennosides : en moyenne supérieur à 2,6 % dans les feuilles). A ébullition sous reflux, on soutire, on essore, on concentre sous vide l'extrait obtenu de façon à obtenir des extraits dont le rapport : poids de plante/extrait concentré varie de 1/1 à 1/8. Par dosage des sennosides lors des différentes phases de concentration, on s'assure que l'on observe aucune dégradation desdits principes actifs, leur quantité étant en proportion avec les rapports de concentration au long du procédé.

On prépare 2 supports microgranulés de façon à obtenir une surface spécifique supérieure à 0,5 m² /gramme par nébulisation (lors de la nébulisation il peut être nécessaire d'effectuer une microgranulation)
- 1er support obtenu par atomisation d'une solution d'inuline à 95 % (on peut utiliser un support inuline prêt à l'emploi obtenu de la même façon : RAFTILINE GR (ORAFTI) avec une granulométrie moyenne de 0,30 mm)
- 2ème support obtenu par atomisation d'une solution d'oligofructose à 95 % (on peut utiliser un support oligofructose prêt à l'emploi obtenu de la même façon : RAFTILOSE P 95 (ORAFTI) avec une granulométrie moyenne de 0,30 mm).

Ces microgranulés sont placés à raison de 75 % inuline et 25 % oligofructose dans un mélangeur à température ambiante. On effectue la granulation des microgranulés avec le mélange d'extraits (A + B : ce mélange A+B est composé de 30 % de A et 70 % de B), à raison de 50 % de la masse, soit 50 ml de A+B pour 100 g de microgranulés.

Le microgranulé ainsi imprégné est mis à sècher en étuve à 40 °C. On obtient après séchage des microgranulés porteurs de l'extrait sec de couleur verte. Puis on calibre de façon à rétablir la granulométrie de départ.

On obtient donc des microgranulés d'inuline et d'oligofructose, dont les cavités internes superficielles sont recouvertes de l'extrait sec de la solution alcoolique obtenue par macération, et de l'extrait aqueux concentré de SENNE.

On recouvre les microgranulés de la même façon et avec le même composant filmogène qu'à l'exemple N°1.

Les microgranulés ainsi obtenus, permettent de reconstituer une forme à dissolution instantanée sans dépôt pour préparation de boisson à visée laxative. On obtient donc une forme galénique équivalente à une tisane effectuée avec les mêmes espèces végétales, et qui comporte les avantages suivants :
Dissolution instantanée
Préparation de la boisson : Eau froide ou Eau chaude, conservant les mêmes principes actifs en quantité et en qualité
Présentation commerciale attractive

### EXEMPLE N°3 : Lait solaire

On répète l'exemple n°1, en utilisant non pas un extrait polyvégétal, mais une macération alcoolique à 80° de centella asiatica, possédant des propriétés cicatrisantes et stimulantes. La fabrication de l'extrait se fait avec de l'alcool éthylique à 80°, qui est spécifique pour l'extraction des dérivés terpéniques responsables de l'activité pharmacologique. La forme extrait alcoolique telle que est difficile à incorporer dans une émulsion, et son utilisation en usage local tel que ou dans la forme émulsion présente des phénomènes algiques importants.

On utilise les mêmes proportions des constituants qu'à l'exemple n°1.

On obtient de la sorte des microgranulés de couleur verte, possédant les principes actifs (dérivés triterpéniques) et ainsi débarassés du solvant d'extraction; donc de ses inconvénients sur la formulation et sur l'utilisation en usage local.

L'enrobage par film est effectué de la même façon qu'à l'exemple N°1.

Ces microgranulés pourront être incorporés dans une émulsion à visée dermatologique restructurante pour la peau, sous forme d'un lait solaire, de formule :

| PHASE GRASSE : | |
|---|---|
| Emulgateur B6 ® | 5 % |
| huile de paraffine | 8 % |
| isopropyl myristate | 4 % |
| huile essentielle de Meleleuca alternifolia | 2 % |
| huile essentielle de Rosa Rubiginosa | 2,50 % |

| PHASE AQUEUSE | |
|---|---|
| conservateur Fondix | 1 % |
| au Q.S.P | 100 % |

On chauffe les deux phases à 65 °C, qui sont incorporées dans un mélangeur émulseur. A 50°C, on réalise l'émulsion au sein du mélangeur en incorporant les microgranulés précédemment préparés dans une proportion allant de 1 à 20 % selon la teneur en dérivés triterpéniques à obtenir. Les microgranulés se trouvent dissous instantanément, les différents principes actifs se répartissant alors indépendamment dans les phases de l'émulsion selon leur affinité chimique.

On obtient un lait solaire avec une teneur garantie en principes actifs, sans incorporer le solvant spécifique qui à été éliminé lors de la préparation du microgranulé.

### EXEMPLE N° 4 : Lait solaire

On répète l'exemple N°3 en utilisant comme extrait, une macération alcoolique à 80° de calendula officinalis, permettant l'extraction optimum des composés : flavonoïdes, saponines, caroténoïdes, qui confèrent au calendula officinalis des propriétés adoucissantes, antiseptiques, anti-inflammatoires.

Les microgranulés absorbés par l'extrait de Calendula Officinalis sont incorporés de la même façon dans une formule de lait, idem exemple n°3.

### EXEMPLE N°5 : Microgranulés porteurs d'Huiles Essentielles

On prépare une solution d'huiles Essentielles dans de l'éthanol à 95° à raison de :

| | |
|---|---|
| Huile Essentielle de Lavande | 6,0 % |
| Huile Essentielle de Romarin | 1,5 % |
| Huile Essentielle de thym | 1,0 % |

On prépare 2 supports microgranulés de façon à obtenir une surface spécifique supérieure à 0,5 m²/gramme par nébulisation (lors de la nébulisation, il peut être nécessaire d'effectuer une microgranulation) :
- 1er support obtenu par atomisation d'une solution d'inuline à 95 % (on peut utiliser un support inuline prêt à l'emploi obtenu de la même façon : RAFTILINE GR (ORAFTI) avec une granulométrie moyenne de 0,30 mm)
- 2ème support obtenu par atomisation d'une solution d'oligofructose à 95 % (on peut utiliser un support oligofructose prêt à l'emploi obtenu de la même façon : ACTILIGHT(BEGHIN SAY) avec une granulométrie moyenne de 0,30 mm).

Ces microgranulés à 75 % inuline et 25 % oligofructose, sont traités de façon identique que les exemples 1 à 3, à l'aide de la solution à raison de 50 % de volume pour 100 % de masse de microgranulés, et enrobés par l'agent filmogène comme les exemples précédents. On obtient des microgranulés à forte odeur aromatique.

On compare, qualitativement par Chromatographie en Phase Gazeuse (C.P.G.), la présence des dérivés terpéniques (traceurs) des huiles essentielles de départ présentes dans la solution à celles présentes dans les microgranulés (les huiles essentielles de 10 grammes de microgranulés sont extraites par 15 ml de méthanol). Les dérivés terpéniques choisis pour l'analyse qualitative en C.P.G. sont du plus volatile au moins volatile :
Alpha Pinène
Béta Pinène
Limonéne
Cinéol
Para Cymène
Camphre
Linalol
Acétate de Linalyl
Alpha Terpinéol
Thymol

Les chromatogrammes de la solution de départ et des microgranulés sont semblables ; on retrouve les éléments les plus volatiles des huiles essentielles.(α Pinène et le β Pinène ) sur les microgranulés démontrant que ce procédé permet de retenir les fractions volatiles des huiles essentielles.

### EXEMPLE N°6 : Microgranulés enrobés , porteurs d'Huile Essentielle de Citron

On prépare une solution à base d'huile essentielle selon :

| | |
|---|---|
| huile essentielle citron | 40 % |
| polyvinyl pyrolidone | 1 % |
| polysorbate 60 | 1 % |
| éthanol à 96° Q.S.P. | 100 % |

On répète l'exemple n°5, on obtient des microgranules de couleur jaune pâle et d'odeur fortement aromatique .

Ces microgranulés subissent une opération d'enrobage avec une substance filmogène (l'acétophtalate de cellulose). La solution d'enrobage se compose :

| | |
|---|---|
| acétophtalate de Cellulose | 10 % |
| acétone | 20 % |
| alcool Ethylique | 70 % |

L'enrobage se fait par pulvérisation à la turbine par procédé conventionnel.

On obtient un microgranulé, soluble à un pH > 5,8 qui se solubilise instantanément dans l'eau, et qui peut ainsi s'incorporer dans différentes formulations pour aromatisation :
Préparation pour boissons
Formes sèches : aromatisation de comprimés , poudres
Tisanes
Etc ...

### EXEMPLE N°7 : Microgranulés enrobés par film gastrorésistant, porteurs d'huile essentielle de thym.

On répète l'exemple n°5 avec une solution à base d'huile essentielle de thym. Les microgranulés sont ensuite enrobés par le même procédé qu'à exemple n°5, avec la solution de polymère suivante :

| | |
|---|---|
| phtalate d'hydroxypropyl de cellullose HP 50® | 10 % |
| isopropanol | 45 % |
| acétate d'éthyle | 45 % |

Les microgranulés obtenus après enrobage présentent un pH > 5,0 d'où leur incorporation dans des formes per-os, en gélules.

### EXEMPLE N°8 : Microgranulés à enrobage insoluble à incorporer dans un gel amincissant.

On répète l'exemple N°7 avec la solution suivante :

| | |
|---|---|
| extrait alcoolique de guarana titré en cafeïne | 20 % |
| extrait alcoolique de lierre | 20 % |
| extrait alcoolique de fucus | 20 % |
| alcool éthylique 95° Q.S.P | 100 % |

On procède à la fabrication des microgranulés absorbés par incorporation de 50 % de la solution d'extrait sur le support microgranulé obtenu comme à l'exemple n° 1, et comprenant :
- 1er support obtenu par atomisation d'une solution d'inuline à 95 % (on peut utiliser un support inuline prêt à l'emploi obtenu de la même façon : RAFTILINE GR (ORAFTI) avec une granulométrie moyenne de 0,30 mm)
- 2 ème support obtenu par atomisation d'une solution de sorbitol (on peut utiliser un support sorbitol prêt à l'emploi obtenu de la même façon : SORBITOL INSTANT (MERCK) avec une granulométrie moyenne de 0,50 mm) ;
- 3 ème support (vitamine E - MICROGRANULES MERCK), obtenu par atomisation.

Les microgranulés sont enrobés par le même procédé qu'à l'exemple n°7, avec la solution de polymère insoluble suivante :

| | |
|---|---|
| éthyl cellulose | 10 % |
| diéthyl phtalate | 2 % |
| alcool éthylique 95° | 44 % |
| chlorure de méthylène | 44 % |

Ces microgranules sont incorporés dans un gel de résine acrylique classique connue comme CARBOPOL 940® , a raison de 2 à 10%, de façon à obtenir un gel de massage où les microgranules sont porteurs d'actifs protégés par un enrobage par film de polymères, ces actifs étant libérés par le frottement dû au massage, qui rompt le film et dissous les actifs qui se libèrent.

### EXEMPLE N°9 : Microgranulés à usage boisson coupe-faim minceur

On répète l'exemple N°8 avec la suspension suivante :

| | |
|---|---|
| extrait sec de garcinia cambodgia | 30 % |
| extrait sec de gymnéma sylvestris | 15 % |
| chitine | 15 % |
| alcool éthylique 95° Q.S.P. | 100 % |

On procède à la fabrication des microgranulés absorbés par incorporation de 20 % de la solution d'extrait sur le support microgranulé obtenu comme à l'exemple n°1, et comprenant :
- 1er support ( 63 %) obtenu par atomisation d'une solution d'inuline à 95 % (on peut utiliser un support inuline prêt à l'emploi obtenu de la même façon : RAFTILINE GR (ORAFTI) avec une granulométrie moyenne de 0,30 mm) ;
- 2 ème support (25 %) : polysaccharide (glucomannane), extrait de la racine d'Amorphophalus konjac.

On obtient donc des microgranulés d'inuline, dont les cavités internes et la surface externe sont recouvertes des extraits secs et de la chitine de la suspension alcoolique, et du glucomananne qui est incorporé lors de la granulation, adhérant à l'intérieur des cavités internes et à la surface du microgranulé.

## Revendications

1. Procédé pour la préparation d'un extrait d'au moins un principe actif sous la forme de granulés secs entièrement solubles en solvant aqueux, qui consiste :
• tout d'abord, à préparer un extrait de principe actif dans un milieu solvant ;
• puis, à déposer cet extrait sur les parois internes et externes d'un support microporeux non soluble dans le milieu solvant, se présentant sous forme de microgranules constitués d'une fibre alimentaire soluble, à base d'un polymère choisi dans le groupe comprenant l'inuline et les oligofructoses ou fructo-oligosaccharides, seuls ou en mélange, lesdits microgranules ayant une surface spécifique d'au moins 0,50 m²/g;
• et à sécher le support granulé imprégné obtenu ;
• puis, à recouvrir les parois imprégnées du support microporeux par un polymère filmogène.

2. Procédé selon la revendication 1, **caractérisé en ce que** le support microporeux se présente sous forme atomisée.

3. Procédé selon l'une des revendications précédentes, **caractérisé en ce que** les fibres alimentaires solubles sont associées à d'autres substances choisies dans le groupe comprenant les polyalcools, les polyols ou les corps gras.

4. Procédé selon la revendication 1, **caractérisé en ce que** le principe actif est un extrait de plantes ou de partie de plantes.

5. Procédé selon la revendication 1, **caractérisé en ce que** le milieu solvant est constitué par un solvant choisi dans le groupe comprenant l'eau, l'éthanol, l'acétone et le dichlorométhane.

6. Procédé selon la revendication 1, **caractérisé en ce que** le support microgranulé obtenu est une poudre à base d'inuline obtenue par extraction à partir de racines de chicorée ou de tout autre végétal riche en inuline.

7. Procédé selon l'une des revendications 1 à 5, **caractérisé en ce que** le support microgranulé est une poudre à base d'oligofructoses obtenus par hydrolyse de l'inuline.

8. Procédé selon l'une des revendications 1 à 5, **caractérisé en ce que** le support microgranulé est une poudre à base d'oligofructoses obtenus par synthèse.

9. Procédé selon la revendication 1, **caractérisée en ce que** le dépôt de l'extrait sur les parois est effectué par granulation humide, à raison de 1% à 60 % en volume par rapport à 100 % de poids desdits microgranulés support, de manière à recouvrir les structures internes par les actifs présents dans la solution, le séchage étant effectué à l'air chaud.

10. Procédé selon la revendication 1, **caractérisé en ce que** le polymère filmogène est un composé soluble dans le système digestif choisi dans le groupe des composés gastro-solubles pH acide ou des composés gastro-resistants pH basique.

11. Procédé selon la revendication 7, **caractérisé en ce que** le polymère filmogène est pulvérisé en turbine ou en lit d'air fluidisé.

## Patentansprüche

1. Verfahren zur Herstellung eines Extrakts aus mindestens einem Grundwirkstoff in Form von in wässrigem Lösungsmittel vollkommen löslichem Trockengranulat, das darin besteht:
• erstens einen Grundwirkstoffextrakt in einem lösenden Medium herzustellen;
• dann diesen Extrakt auf die Innen- und Aussenwandungen eines mikroporösen Trägers aufzubringen, der in dem lösenden Medium nicht löslich ist, und der in der Form von aus einer Nahrungsfaser bestehenden Feinstkörnchen auf der Basis eines Polymers vorliegt, das aus der Gruppe ausgewählt ist, die Inulin und Oligofruktosen oder Fruktooligosaccharide allein oder im Gemisch enthält, wobei diese Feinstkörnchen eine spezifische Oberfläche von mindestens 0,50 m²/g haben;
• den erhaltenen durchtränkten Granulatträger zu trocknen;
• dann die durchtränkten Wandungen des mikroporösen Trägers mit einem filmbildenden Polymer zu überziehen.

2. Verfahren nach Anspruch 1, **dadurch gekennzeichnet, dass** der mikroporöse Träger in zerstäubter Form vorliegt.

3. Verfahren nach einem der vorhergehenden Ansprüche, **dadurch gekennzeichnet, dass** die löslichen Nahrungsfasern an weitere Stoffe gebunden sind, die aus der Gruppe ausgewählt sind, die Polyalkohole, Polyole oder Fettstoffe umfassen.

4. Verfahren nach Anspruch 1, **dadurch gekennzeichnet, dass** der Grundwirkstoff ein Extrakt aus Pflanzen oder Pflanzenteilen ist.

5. Verfahren nach Anspruch 1, **dadurch gekennzeichnet, dass** das lösende Medium aus einem Lösungsmittel besteht, das aus der Gruppe ausgewählt ist, die Wasser, Ethanol, Aceton und Dichlormethan umfasst.

6. Verfahren nach Anspruch 1, **dadurch gekennzeichnet, dass** der Mikrogranulatträger ein Pulver auf der Basis von Inulin ist, das aus Chicoreewurzeln oder einem anderen inulinreichen Gemüse extrahiert ist.

7. Verfahren nach einem der Ansprüche 1 bis 5, **dadurch gekennzeichnet, dass** der Mikrogranulatträger ein Pulver auf der Basis von Oligofruktosen ist, die durch Hydrolyse des Inulins gewonnen werden.

8. Verfahren nach einem der Ansprüche 1 bis 5, **dadurch gekennzeichnet, dass** der Mikrogranulatträger ein Pulver auf der Basis von Oligofruktosen ist, die durch Synthese gewonnen werden.

9. Verfahren nach Anspruch 1, **dadurch gekennzeichnet, dass** das Aufbringen des Extrakts auf die Wandungen durch Feuchtgranulation im Verhältnis 1 Vol.-% bis 60 Vol.-% zu 100 Gew.-% dieses Trägermikrogranulats bewerkstelligt wird, um die Innenstrukturen mit den in der Lösung vorhandenen Wirkstoffen zu überziehen, wobei das Trocknen mit Warmluft erfolgt.

10. Verfahren nach Anspruch 1, **dadurch gekennzeichnet, dass** das filmbildende Polymer eine im Verdauungssystem lösliche Verbindung ist, die aus der Gruppe der im Magen löslichen pH-sauren Verbindungen oder der im Magen nicht löslichen pH-basischen Verbindungen ausgewählt ist.

11. Verfahren nach Anspruch 7, **dadurch gekennzeichnet, dass** das filmbildende Polymer in einer Turbine oder einem Wirbelbett pulverisiert wird.

## Claims

1. Method for the preparation of an extract of at least one active principle in the form of completely soluble dry granules in an aqueous solvent, which consists:
• first, in preparing an extract of an active principle in a solvent medium;
• then, in depositing this extract on the internal and external walls of a microporous carrier insoluble in the solvent medium, provided in the form of microgranules constituted by a dietary soluble fibre based on a polymer selected from the group comprising inulin and oligofructoses and fructo-oligosaccharides, on their own or mixed, having a specific surface area of at least 0.50 m²/g;
• and in drying the impregnated granular carrier obtained;
• then, in coating the impregnated walls of the microporous carrier with a film-forming polymer.

2. Method according to Claim 1, **characterized in that** the microporous carrier is provided in spray-dried form.

3. Method according to one of the preceding claims, **characterized in that** the soluble dietary fibres are combined with other substances selected from the group comprising polyalcohols, polyols and fatty substances.

4. Method according to Claim 1, **characterized in that** the active principle is an extract of plants or of a portion of plants.

5. Method according to Claim 1, **characterized in that** the solvent medium consists of a solvent selected from the group comprising water, ethanol, acetone and dichloromethane.

6. Method according to Claim 1, **characterized in that** the microgranular carrier obtained is a powder based on inulin obtained by extraction from chicory roots or from any other inulin-rich plant.

7. Method according to one of Claims 1 to 5, **characterized in that** the microgranular carrier is a powder based on oligofructoses obtained by hydrolysis of inulin.

8. Method according to one of Claims 1 to 5, **characterized in that** the microgranular carrier is a powder based on oligofructoses obtained by synthesis.

9. Method according to Claim 1, **characterized in that** the deposition of the extract on the walls is carried out by wet granulation, at the rate of 1% to 60% by volume relative to 100% by weight of the said carrier microgranules, so as to coat the internal structures with the active agents present in the solution, the drying being carried out with hot air.

10. Method according to Claim 1, **characterized in that** the film-forming polymer is a compound soluble in the digestive system selected from the group of acidic pH, gastro-soluble compounds or basic pH, gastro-resistant compounds.

11. Method according to Claim 7, **characterized in that** the film-forming polymer is pulverized in a turbine or in a fluidized bed.
